# EUROPÄISCHE PATENTSCHRIFT

(11) **EP 1 737 834 B1**
(45) Veröffentlichungstag und Bekanntmachung des Hinweises auf die Patenterteilung: **03.10.2007**
(21) Anmeldenummer: 05731325.6
(22) Anmeldetag: 08.04.2005
(51) Int. Cl.: C07D 251/70

(54) **VERFAHREN ZUR HERSTELLUNG VON 1,3,5-TRIAZINCARBAMATEN UND -HARNSTOFFEN**
METHOD FOR PRODUCING 1,3,5-TRIAZINE CARBAMATES AND UREAS
PROCEDE POUR PRODUIRE DES CARBAMATES ET DES UREES DE 1,3,5-TRIAZINE

(30) Priorität: 14.04.2004 DE 102004018544
(43) Veröffentlichungstag der Anmeldung: 03.01.2007
(73) Patentinhaber: BASF Aktiengesellschaft, 67056 Ludwigshafen (DE)
(72) Erfinder: HEISCHKEL, Yvonne, 68199 Mannheim (DE); WAGNER, Eva, 67346 Speyer (DE); SCHNEIDER, Jörg, 69469 Weinheim (DE); SCHWALM, Reinhold, 67157 Wachenheim (DE); SCHERR, Günter, 67065 Ludwigshafen (DE); HAEBERLE, Karl, 67346 Speyer (DE)
(86) Internationale Anmeldenummer: PCT/EP2005/003690
(87) Internationale Veröffentlichungsnummer: WO 2005/100328

(56) Entgegenhaltungen:
- DE-A1- 10 151 564

## Beschreibung

Die vorliegende Erfindung beschreibt ein Verfahren zur Herstellung von 1,3,5-Triazincarbamaten und -harnstoffen aus anderen 1,3,5-Triazincarbamaten.

US 4,939,213 beschreibt die Beschichtungen, die durch Härtung von Tricarbamoyltriazinen mit aktive Wasserstoffatome enthaltenden Polyacrylaten oder Polyesterolen bei Raumtemperatur in Gegenwart eines Härtungskatalysators entstehen. Als Katalysator werden Zinnsalze sowie quaternäre und ternäre kationische Salze offenbart. In den Beispielen 7 und 8 wird die Härtung bei Raumtemperatur in Gegenwart eines Zinnkatalysators beschrieben, jedoch ohne die Angabe der Reaktionszeit.

US 5,565,243 beschreibt die Grundierungslacke, die Tricarbamoyltriazine mit Alkylgruppen bis zu 12 Kohlenstoffatomen und Harz als Bindemittel sowie einen Decklack aus Polyepoxid und Vernetzer enthalten, wobei der Grundierungslack beispielsweise mit Zinnverbindungen und der Decklack beispielsweise mit sekundären oder tertiären Aminen gehärtet werden kann. Die Beispiele werden jeweils bei Raumtemperatur gehärtet.

Nachteilig an diesen Systemen ist, daß der abgespaltene niedere Alkohol aus den Tricarbamoyltriazinen nicht entweichen kann. Zudem kann durch die Härtung infolge zunehmender Vernetzung in der Beschichtung kein hoher Umsatz erzielt werden. Die Härtung bei Raumtemperatur erfordert zudem lange Reaktionszeiten. Die eingesetzten Tricarbamoyltriazine werden durch Umsetzung von hochreaktiven 1,3,5-Triazintriisocyanat mit Alkoholen, Aminen etc. hergestellt, die infolge ihrer Reaktivität schlecht lager- und transportierbar und darüberhinaus toxisch sind.

Die Darstellung von 1,3,5-Triazincarbamaten ist beispielsweise in DE-A1 101 51 564 oder WO 97/08235, S. 3, Z. 9 - 22 beschrieben. Die dort genannten Darstellungswege führen zu alkylsubstituierten 1,3,5-Triazincarbamaten. Diese Methoden sind aufgrund des stark basischen Reaktionsmediums nicht für funktionelle Gruppen wie Ester- oder Carbamatgruppen geeignet.

EP-A2 305 115 beschreibt strahlungsaktivierbare 1,3,5-Triazinverbindungen, die mindestens eine halogenhaltige Gruppe CX₃ enthalten und über UV-Belichtung eine radikalische Polymerisation photochemisch auslösen können. Weiterhin können die Triazinverbindungen radikalisch polymerisierbare Gruppen, z. B. Hydroxyethylacrylat, angebunden über eine Urethangruppe, enthalten.

EP-A 359 430 beschreibt ebenfalls halogenhaltige 1,3,5-Triazinverbindungen, die gleichzeitig eine radikalisch polymerisierbare Gruppe enthält. Derartige Verbindungen bilden unter dem Einfluss von Licht Radikale.

Die strahlungsaktivierbaren Halogengruppen in diesen Systemen wirken sich nachteilig auf die UV-Beständigkeit solcher Verbindungen oder Beschichtungen, die solche enthalten, aus und führen zu einer erhöhten Vergilbung.

In EP-A 366 884 sind 1,3,5-Triazinverbindungen beschrieben, die mindestens zwei vinyl-terminierte Gruppen und mindestens eine Carbamylgruppe enthalten. Diese 1,3,5-Triazinverbindungen enthalten Reaktionsprodukte von Melamin mit Aldehyden, insbesondere mit Formaldehyd. Neben den Vinylendgruppen enthalten die 1,3,5-Triazinverbindungen Methylol- und/oder alkylierte Methylolgruppen.

Ein ähnliches System ist in EP-A 473 948 beschrieben. Es handelt sich um substituierte 1,3,5-Triazine, die durch Kondensation von Melamin mit Formaldehyd erhalten werden und ethylenisch ungesättigte Gruppen enthalten. Derartige Gruppen sind säureempfindlich.

Aufgabe der vorliegenden Erfindung war es ein Verfahren zur Herstellung von 1,3,5-Triazincarbamaten und -harnstoffen zur Verfügung zu stellen, das von un- oder wenig toxischen Verbindungen ausgehen soll und die Zielprodukte in hohen Ausbeuten bei niedrigen Reaktionszeiten liefern soll.

Die Aufgabe wird gelöst durch ein Verfahren zur Herstellung von 1,3,5-Triazincarbamaten der Formel (1), 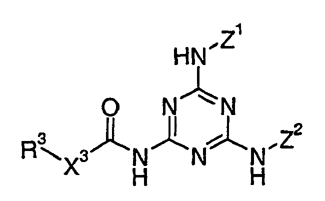 aus 1,3,5-Triazincarbamaten der Formel (II), 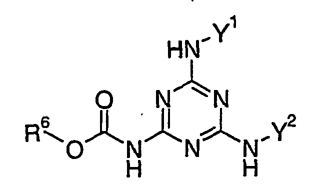 worin
Y¹ und Z¹ entweder beide Wasserstoff oder Y¹ eine Gruppe der Formel -(CO)-O-R⁴ und Z¹ eine Gruppe der Formel -(CO)-X¹-R¹,
Y² und Z² entweder beide Wasserstoff oder Y² eine Gruppe der Formel -(CO)-O-R⁵ und Z² eine Gruppe der Formel -(CO)-X²-R²,
R¹, R², R³, R⁴, R⁵ und R⁶ jeweils unabhängig voneinander den Rest eines Alkohols oder Amins und
X¹, X² und X³ jeweils unabhängig voneinander Sauerstoff oder unsubstituierten Stickstoff (NH)
bedeuten,
in dem
man die Reaktion bei einer Temperatur von 40 bis 120 °C und
in Gegenwart mindestens eines Katalysators durchführt, der ausgewählt ist aus der Gruppe umfassend Zinnverbindungen, Cäsiumsalze, Alkalimetall(hydrogen)carbonate und tertiäre Amine.

Das erfindungsgemäße Verfahren liefert 1,3,5-Triazincarbamate und -harnstoffe in gegenüber dem Stand der Technik verbesserten Ausbeuten, so daß insbesondere die Raum-Zeit-Ausbeute bei der Herstellung verbessert ist. Durch die Erhöhung der Temperatur gegenüber der Härtung bei Raumtemperatur und die erfindungsgemäße Verfahrensführung werden Umsätze erzielt und damit Verbindungen darstellbar, die durch Umsetzung bei Raumtemperatur nicht zu erzielen wären.

Da die Temperatur im erfindungsgemäßen katalysierten Verfahren niedriger ist als bei der rein thermischen Herstellung, sind günstigere Farbzahlen erzielbar.

Die Reste R⁴, R⁵ und R⁶ sind jeweils unabhängig voneinander abgeleitet von Alkoholen R⁴OH, R⁵OH und R⁶OH, die einen Siedepunkt bei Normaldruck von 120 °C oder weniger aufweisen, bevorzugt von 100 °C oder weniger, besonders bevorzugt von 80 °C oder weniger und ganz besonders bevorzugt von 70 °C oder weniger.

Beispiele für die Reste R⁴, R⁵ und R⁶ sind jeweils unabhängig voneinander C₁-C₄-Alkyl, womit in dieser Schrift Methyl, Ethyl, *iso*-Propyl, n-Propyl, n-Butyl, *iso*-Butyl, *sek*-Butyl und *tert*-Butyl gemeint sind, bevorzugt sind Methyl, Ethyl und n-Butyl, ganz besonders bevorzugt sind Methyl und n-Butyl und insbesondere Methyl.

Die Reste R⁴, R⁵ und R⁶ können gleich oder verschieden sein, bevorzugt handelt es sich um nicht mehr als zwei verschiedene Reste.

Bevorzugte Verbindungen (II) sind solche, in denen mindestens einer, besonders bevorzugt zwei der Reste Y¹ und Y² die Gruppen -(CO)-O-R⁴ bzw. -(CO)-O-R⁵ ist/sind. Demzufolge sind bevorzugte Verbindungen (I) solche, in denen mindestens einer, besonders bevorzugt zwei der Reste Z¹ und Z² die Gruppen -(CO)-X¹-R¹ bzw. -(CO)-X²-R² ist/sind.

Ganz besonders bevorzugt eingesetzte 1,3,5-Triazincarbamate (II) sind die 1,3,5-Triazin(trimethyl)carbamate, 1,3,5-Triazin(triethyl)carbamate, 1,3,5-Triazin(tri-n-butyl)carbamate oder gemischte Methyl/n-Butyl-1,3,5-Triazincarbamate.

Die Darstellung der eingesetzten 1,3,5-Triazincarbamate ist erfindungsgemäß nicht wesentlich und kann beispielsweise erfolgen wie in DE-A1 101 51 564 oder WO 97/08235, S. 3, Z. 9 - 22 beschrieben.

Die Reste R¹-X¹, R²-X² und R³-X³ sind abgeleitet von Alkoholen R¹OH, R²OH und R³OH bzw. Aminen R¹NH₂, R²NH₂ und R³NH₂.

Besonders bevorzugt im erfindungsgemäßen Verfahren sind solche Alkohole R¹OH, R²OH und R³OH bzw. Amine R¹NH₂, R²NH₂ und R³NH₂, deren niedrigst siedendes eine Siedepunktsdifferenz von mindestens 20 °C, bevorzugt mindestens 40 und besonders bevorzugt mindestens 60 °C gegenüber dem höchstsiedenden der Alkohole R⁴OH, R⁵OH und R⁶OH aufweisen.

Bei den Resten R¹, R² und R³ kann es sich beispielsweise handeln um C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, sowie ferner um Reste

-(CO)-R⁷, -(CO)-O-R⁷ oder -(CO)-(NH)-R⁷,

worin R⁷ C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können bedeuten kann.

Darin bedeuten C₁ - C₁₈-Alkyl und gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl beispielsweise gegebenenfalls durch Aryl, Alkyl, Aryloxy, Alkyloxy, Heteroatome und/oder Heterocyclen substituiertes C₁ - C₁₈-Alkyl beispielsweise Methyl, Ethyl, Propyl, Isopropyl, n-Butyl, sec-Butyl, tert.-Butyl, Pentyl, Hexyl, Heptyl, Octyl, 2-Etylhexyl, 2,4,4-Trimethylpentyl, Decyl, Dodecyl, Tetradecyl, Heptadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl, 1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonylethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, 1,3-Dioxolan-2-yl, 1,3-Dioxan-2-yl, 2-Methyl-1,3-dioxolan-2-yl, 4-Methyl-1,3-dioxolan-2-yl, 2-lsopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, Butylthiomethyl, 2-Dodecylthioethyl, 2-Phenylthioethyl, 2,2,2-Trifluorethyl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methylaminopropyl, 4-Methylaminobutyl, 6-Methylaminohexyl, 2-Dimethylaminoethyl, 2-Dimethylaminopropyl, 3-Dimethylaminopropyl, 4-Dimethylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxy-hexyl, 2-Methoxyethyl, 2-Methoxypropyl, 3-Methoxypropyl,-4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl,
gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl beispielsweise 5-Hydroxy-3-oxa-pentyl, 8-Hydroxy-3,6-dioxa-octyl, 11-Hydroxy-3,6,9-trioxa-undecyl, 7-Hydroxy-4-oxä-heptyl, 11-Hydroxy-4,8-dioxa-undecyl, 15-Hydroxy-4,8,12-trioxa-pentadecyl, 9-Hydroxy-5-oxa-nonyl, 14-Hydroxy-5,10-oxa-tetradecyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dioxa-undecyl, 15-Methoxy-4,8,12-trioxa-pentadecyl, 9-Methoxy-5-oxa-nonyl, 14-Methoxy-5,10-oxa-tetradecyl, 5-Ethoxy-3-oxa-pentyl, 8-Ethoxy-3,6-dioxa-octyl, 11-Ethoxy-3,6,9-trioxa-undecyl, 7-Ethoxy-4-oxa-heptyl, 11-Ethoxy-4,8-dioxa-undecyl, 15-Ethoxy-4,8,12-trioxa-pentadecyl, 9-Ethoxy-5-oxa-nonyl oder 14-Ethoxy-5,10-oxa-tetradecyl.

Die Anzahl der Sauerstoff- und/oder Schwefelatome und/oder Iminogruppen ist nicht beschränkt. In der Regel beträgt sie nicht mehr als 5 in dem Rest, bevorzugt nicht mehr als 4 und ganz besonders bevorzugt nicht mehr als 3.

Weiterhin befinden sich zwischen zwei Heteroatomen in der Regel mindestens ein Kohlenstoffatom, bevorzugt mindestens zwei.

Substituierte und unsubstituierte Iminogruppen können beispielsweise Imino-, Methylimino-, *iso*-Propylimino, n-Butylimino oder tert-Butylimino sein.

Weiterhin bedeutet
gegebenenfalls durch Aryl, Alkyl, Aryloxy, Alkyloxy, Heteroatome und/oder Heterocyclen substituiertes C₂- C₁₈-Alkenyl beispielsweise Vinyl, 1-Propenyl, Allyl, Methallyl, 1,1-Dimethylallyl, 2-Butenyl, 2-Hexenyl, Octenyl, Undecenyl, Dodecenyl, Octadecenyl, 2-Phenylvinyl, 2-Methoxyvinyl, 2-Ethoxyvinyl, 2-Methoxyallyl, 3-Methoxyallyl, 2-Ethoxyallyl, 3-Ethoxyallyl oder 1- oder 2-Chlorvinyl,
gegebenenfalls durch Aryl, Alkyl, Aryloxy, Alkyloxy, Heteroatome und/oder Heterocyclen substituiertes C₆ - C₁₂-Aryl beispielsweise Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, *iso*-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxymethylphenyl,
gegebenenfalls durch Aryl, Alkyl, Aryloxy, Alkyloxy, Heteroatome und/oder Heterocyclen substituiertes C₅ - C₁₂-Cycloalkyl beispielsweise Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl sowie ein gesättigtes oder ungesättigtes bicyclisches System wie z.B. Norbornyl oder Norbornenyl und
ein fünf- bis sechsgliedriger, Sauerstoff-, Stickstoff- und/oder Schwefelatome aufweisender Heterocyclus beispielsweise Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, lsopropylthiophenyl oder tert.-Butylthiophenyl.

In einer Ausführungsform der Erfindung kann es sich bei den Alkoholen R¹OH, R²OH und R³OH um Monoole handeln, also solche Alkohole mit genau einer Hydroxyfunktion (-OH).

Bevorzugte Monoole R¹OH, R²OH und R³OH sind n-Butanol, sek-Butanol, iso-Butanol, tert.-Butanol, n-Pentanol, n-Hexanol, n-Heptanol, n-Octanol, n-Decanol, 2-Ethylhexanol, Ethylenglykolmonomethylether, Ethylenglykolmonoethylether, 1,3-Propandiolmonomethylether, Laurylalkohol (1-Dodecanol), Myristylalkohol (1-Tetradecanol), Cetylalkohol (1-Hexadecanol), Stearylalkohol (1-Octadecanol), 9-cis-Octadecen-1-ol (Oleylalkohol), 9-trans-Octadecen-1-ol 9-cis-Octadecen-1,12-diol (Ricinolalkohol), all-cis-9,12-Octadecadien-1-ol (Linoleylalkohol), all-cis-9,12,15-Octadecatrien-1-ol (Linolenylalkohol), 1-Eicosanol (Arachidylalkohol), 9-cis-Eicosen-1-ol (Gadoleylalkohol), 1-Docosanol (Behenylalkohol), 1,3-cis-Docosen-1-ol, 1,3-trans-Docosen-1-ol (Brassidylalkohol), Cyclopent-2-en-1-ol, Cyclopent-3-en-1-ol, Cyclohex-2-en-1-ol oder Allylalkohol.

Weiterhin kann es sich um alkoxylierte Monoole der Formel

R⁸-O-[-Xᵢ-]ₙ-H

handeln, worin
R⁸ C₁ - C₁₈-Alkyl, bevorzugt C₁-C₄-Alkyl sein kann,
n eine positive ganze Zahl zwischen 1 und 50, bevorzugt zwischen 1 und 30, besonders bevorzugt zwischen 1 und 20 und ganz besonders bevorzugt zwischen 2 und 10 und
jedes Xᵢ für i = 1 bis n unabhängig voneinander ausgewählt sein kann aus der Gruppe -CH₂-CH₂-O-, -CH₂-CH(CH₃)-O-, -CH(CH₃)-CH₂-O-, -CH₂-C(CH₃)₂-O-, -C(CH₃)₂-CH₂-O-, -CH₂-CHVin-O-, -CHVin-CH₂-O-, -CH₂-CHPh-O- und -CHPh-CH₂-O-, bevorzugt aus der Gruppe -CH₂-CH₂-O-, -CH₂-CH(CH₃)-O- und -CH(CH₃)-CH₂-O-, und besonders bevorzugt -CH₂-CH₂-O-,
worin Ph für Phenyl und Vin für Vinyl steht.

In einer bevorzugten Ausführungsform der vorliegenden Erfindung handelt es sich bei dem Monool um eine Verbindung, die mindestens eine, beispielsweise ein bis drei, bevorzugt ein bis zwei und besonders bevorzugt eine polymerisierbare Gruppe und genau eine Hydroxygruppe trägt.

Polymerisierbare Gruppen sind beispielsweise Vinylether-, Acrylat- oder Methacrylatgruppen, bevorzugt (Meth)Acrylatgruppen und besonders bevorzugt Acrylatgruppen.

Beispiele für Verbindungen, die mindestens eine polymerisierbare Gruppe und genau eine Hydroxygruppe tragen sind solche der Formeln

(III) H₂C=CR⁹-CO-O-R¹⁰-OH,

(IV) H₂C=CR⁹-CO-O-[-Xᵢ-]ₖ-H

oder

(V) H₂C=CH-O-R¹⁰-OH,

worin
R⁹ Wasserstoff oder Methyl, bevorzugt Wasserstoff,
R¹⁰ einen zweiwertigen linearen oder verzweigten C₂-C₁₈-, bevorzugt C₂-C₁₂-, besonders bevorzugt C₂-C₈- und ganz besonders bevorzugt C₂-C₆-Alkylenrest,
Xᵢ die gleiche Bedeutung hat wie oben ausgeführt und
k eine positive ganze Zahl von 1 bis 20, bevorzugt von 2 bis 15, besonders bevorzugt von 2 bis 10 und ganz besonders bevorzugt von 2 bis 5
bedeutet.

Beispiele für R¹⁰ sind 1,2-Ethylen, 1,2- oder 1,3-Propylen, 1,2-, 1,3- oder 1,4-Butylen, 1,6-Hexylen, 1,1-Dimethyl-1,2-ethylen, 1,2-Dimethyl-1,2-ethylen, Phenylethylen, bevorzugt 1,2-Ethylen, 1,2- oder 1,3-Propylen, 1,4-Butylen oder 1,6-Hexylen, besonders bevorzugt 1,2-Ethylen, 1,2-Propylen oder 1,4-Butylen und ganz besonders bevorzugt 1,2-Ethylen.

Bevorzugte Verbindungen, die mindestens eine polymerisierbare Gruppe und genau eine Hydroxygruppe tragen sind Hydroxyethyl(meth)acrylat, Hydroxypropyl(meth)-acrylat, 4-Hydroxybutyl(meth)acrylat, 6-Hydroxyhexyl(meth)acrylat, zwei bis zehnfach, bevorzugt zwei bis fünffach ethoxylierte (Meth)Acrylsäure sowie Pentaerythrittri(meth)-acrylat.

In einer weiteren Ausführungsform der Erfindung kann es sich bei den Alkoholen R¹OH, R²OH und R³OH um Di- oder Polyole handeln, also solche Alkohole mit zwei oder mehr als zwei Hydroxyfunktionen (-OH), bevorzugt 2 bis 6, bevorzugt bevorzugt 2 bis 4, ganz besonders bevorzugt 2 bis 3 und insbesondere 2.

Beispiele für Di- oder Polyole sind 1,2-Propandiol, Ethylenglykol, Diethylenglykol, Triethylenglykol, Tetraethylenglykol, Pentaethylenglykol, 2,2-Dimethyl-1,2-Ethandiol, 1,3-Propandiol, 1,2-Butandiol, 1,3-Butandiol, 1,4-Butandiol, But-2-en-1,4-diol, But-2-in-1,4-diol, Tricyclodecandimethanol, Trimethylolbutan, Trimethylolpropan, Trimethylolethan, Neopentylglykol, Hydroxypivalinsäureneopentylglykolester, Pentaerythrit, 2-Ethyl-1,3-Propandiol, 2-Methyl-1,3-Propandiol, 2-Ethyl-1,3-Hexandiol, 2,4-Diethyloktan-1,3-diol, Glycerin, Ditrimethylolpropan, Dipentaerythrit, Hydrochinon, Bisphenol A, Bisphenol F, Bisphenol B, Bisphenol S, 2,2-Bis(4-hydroxycyclohexyl)propan, 1,1-, 1,2-, 1,3- und 1,4-Cyclohexandimethanol, 1,2-, 1,3- oder 1,4-Cyclohexandiol, Sorbit, Mannit, Diglycerol, Threit, Erythrit, Adonit (Ribit), Arabit (Lyxit), Xylit, Dulcit (Galactit), Maltit, Isomalt, 1,2-, 1,3- oder 1,4-Aminophenol, 1,2-, 1,3- oder 1,4-Bishydroxymethylbenzol, 2-, 3- oder 4-Hydroxybenzoesäure, 2-, 3- oder 4-Aminobenzoesäure, die jeweils gegebenenfalls alkoxyliert, bevorzugt ethoxyliert und/oder propoxyliert und besonders bevorzugt ethoxyliert sein können, Poly-THF mit einer Molmasse zwischen 162 und 2000, Poly-1,3-Propandiol mit einer Molmasse zwischen 134 und 1178 oder Polyethylenglykol mit einer Molmasse zwischen 106 und 1000.

In einer besonderen Ausführungsform kann auch mindestens einer der Alkohole R¹OH, R²OH und R³OH ausgewählt sein unter Polyetherolen oder Polyesterolen unter der Voraussetzung, daß gleichzeitig mindestens einer der Alkohole R¹OH, R²OH und R³OH eines der oben aufgeführten Monoole mit mindestens einer polymerisierbaren Gruppe und genau einer Hydroxygruppe ist.

Als Polyesterole kommen z.B. solche in Betracht, wie sie durch Veresterung von Polycarbonsäuren, vorzugsweise Dicarbonsäuren, mit den oben genannten Polyolen hergestellt werden können.

Die Ausgangsstoffe für solche Polyesterole sind dem Fachmann bekannt. Bevorzugt können als Polycarbonsäuren Oxalsäure, Maleinsäure, Fumarsäure, Bernsteinsäure, Glutarsäure, Adipinsäure, Sebacinsäure, Dodekandisäure, o-Phthalsäure, Isophthalsäure, Terephthalsäure, Trimellithsäure, Azelainsäure, 1,4-Cyclohexandicarbonsäure oder Tetrahydrophthalsäure, deren Isomere und Hydrierungsprodukte sowie veresterbare Derivate, wie Anhydride oder Dialkylester, beispielsweise C₁-C₄-Alkylester, bevorzugt Methyl-, Ethyl- oder n-Butylester, der genannten Säuren eingesetzt werden.

Als Hydroxygruppen tragende Carbonsäuren oder Lactone kommen 4-Hydroxybenzoesäure, 6-Hydroxy-2-naphthalinsäure, Pivalolacton oder ε-Caprolacton in Betracht. Als Polyole kommen die oben genannten mehrfunktionellen Alkohole, vorzugsweise Neopentylglykol, Trimethylolpropan, Trimethylolethan, Pentaerythrit, Dimethylolpropionsäure oder Dimethylolbuttersäure in Betracht.

Das bevorzugte Molekulargewicht der Polyesterole beträgt bis zu 5000 g/mol, besonders bevorzugt bis zu 3000, ganz besonders bevorzugt 500 bis 2000 und insbesondere 500 bis 1500 g/mol.

In einer weiteren Ausführungsform der Erfindung kann es sich bei den Aminen R¹NH₂, R²NH₂ und R³NH₂ um Monoamine handeln, also solche Amine mit genau einer Aminofunktion (-NH₂).

Dies können beispielsweise sein Methylamin, Ethylamin, *iso*-Propylamin, n-Propylamin, n-Butylamin, *iso*-Butylamin, *sek*-Butylamin, *tert*-Butylamin, n-Pentylamin, n-Hexylamin, n-Heptylamin, n-Octylamin, n-Decylamin, n-Dodecylamin, 2-Ethylhexylamin, Stearylamin, Cetylamin oder Laurylamin, ferner Cyclopentylamin, Cyclohexylamin, Cyclooctylamin, Cyclododecylamin, Monoethanolamin, 1,2-Propanolamin, 1,3-Propanolamin, 1,4-Butanolamin, 1,6-Hexanolamin und Aminoethylethanolamin.

Bevorzugt wird die Reaktion in Gegenwart von mindestens einem Monoamin, Monool oder Mischungen aus mindestens einem Monool und mindestens einem Polyol durchgeführt, besonders bevorzugt in Gegenwart von mindestens einem Monool oder Mischungen aus mindestens einem Monool und mindestens einem Polyol und ganz besonders bevorzugt in Gegenwart von genau einem Monool oder einer Mischung aus genau einem Monool und genau einem Polyol.

Der Katalysator, der erfindungsgemäß in der Reaktion eingesetzt wird, ist ausgewählt aus der Gruppe umfassend Zinnverbindungen, Cäsiumsalze, Alkalimetall(hydrogen)carbonate und tertiäre Amine.

Zinnverbindungen sind alle organometallischen Zinnverbindungen, bevorzugt Zinn-(II)-n-octanoat, Zinn-(II)-2-ethyl-I-hexanoat, Zinn-(II)-laurat, Dibutylzinnoxid, Dibutylzinndichlorid, Dibutylzinndiacetat, Dibutylzinndilaurat, Dibutylzinndimaleat oder Dioctylzinndiacetat, besonders bevorzugt sind Zinn(II)-n-octanoat, Zinn-(II)-2-ethyl-I-hexanoat, Zinn-(II)-laurat, Dibutylzinnoxid, Dibutylzinndiacetat, Dibutylzinndilaurat, ganz besonders bevorzugt sind Dibutylzinnoxid, Dibutylzinndiacetat, Dibutylzinndilaurat und insbesondere Dibutylzinndilaurat.

Zinnverbindungen sind jedoch toxikologisch bedenklich und daher erfindungsgemäß weniger bevorzugt, besonders dann, wenn sie in dem Reaktionsgemisch verbleiben. Demgegenüber sind Cäsiumsalze und Alkalimetall(hydrogen)carbonate unbedenklich.

Bevorzugte Cäsiumsalze sind dabei solche, in denen folgende Anionen enthalten sind: F⁻, Cl⁻, ClO⁻, ClO₃⁻, ClO₄⁻, Br⁻, J⁻, JO₃⁻, CN⁻, OCN⁻, NO₂⁻, NO₃⁻, HCO₃⁻, CO₃²⁻, S²⁻, SH⁻, HSO₃⁻, SO₃²⁻, HSO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, (OCₘH₂ₘ₊₁)⁻, (CₘH₂ₘ₋₁O₂)⁻, (CₘH₂ₘ₋₃O₂)⁻ sowie (Cₘ₊₁H₂ₘ₋₂O₄)²⁻, wobei m für die Zahlen 1 bis 20 steht.

Besonders bevorzugt sind dabei Cäsiumcarboxylate, bei denen das Anion den Formeln (CₘH₂ₘ₋₁O₂)⁻ sowie (Cₘ₊₁H₂ₘ₋₂O₄)²⁻ mit m gleich 1 bis 20, gehorcht. Ganz besonders bevorzugte Cäsiumsalze weisen als Anionen Monocarboxylate der allgemeinen Formel (CₘH₂ₘ₋₁O₂)⁻ auf, wobei m für die Zahlen 1 bis 20 steht. Hierbei sind insbesondere zu erwähnen Formiat, Acetat, Propionat, Hexanoat und 2-Ethylhexanoat, ganz besonders bevorzugt ist Cäsium acetat.

Die Cäsiumsalze können dem Ansatz in fester Form, oder gelöster Form zugesetzt werden. Als Lösemittel sind polare, aprotische Lösemittel oder auch protische Lösemittel geeignet. Besonders geeignet sind neben Wasser auch Alkohole; ganz besonders geeignet sind Polyole, wie z.B. Ethan-, Propan-oder Butandiole und Glykolether.

Zur Verbesserung der Löslichkeit der Cäsiumsalze im Reaktionsmedium können diese gegebenenfalls mit Phasentransferkatalysatoren eingesetzt werden. Geeignete Phasentransferkatalysatoren sind beispielsweise Kronenether wie 18-Krone-6 oder Tetraalkylammoniumsalze wie Tetrabutylammoniumbromid.

Alkalimetall(hydrogen)carbonate sind beispielsweise die Carbonate Li₂CO₃, Na₂CO₃ und K₂CO₃ sowie die Hydrogencarbonate LiHCO₃, NaHCO₃ und KHCO₃, bevorzugt sind Na₂CO₃ und K₂CO₃ und besonders bevorzugt K₂CO₃.

Tertiäre Amine sind beispielsweise Trioctylamin, Tridodecylamin, Tribenzylamin, N,N,N',N'-Tetramethylethylendiamin, 1-Methylpyrrol, Pyridin, 4-Dimethylaminopyridin, Picolin, N,N'-Dimethylpiperazin, N-Methylmorpholin, N-Methylpiperidin, N-Ethylpiperidin, N,N-Dimethylanilin, N,N-Dimethylbenzylamin, N-Methylimidazol, 1,4-Diazabicyclo[2.2.2]octan, 1,5-Diazabicyclo[4.3.0]non-5-en oder 1,8-Diazabicyclo[5.4.0]undec-7-en.

Bevorzugte Katalysatoren sind Cäsiumsalze und Alkalimetall(hydrogen)carbonate, besonders bevorzugt sind die Cäsiumsalze.

Weiterhin denkbar wären als Katalysatoren Alkoholate (beispielsweise Natrium- oder Kalium Alkoholate von C₁-C₄-Alkylalkoholen, bevorzugt Natrium- und Kalium- -methanolat und -ethanolat), Hydroxide (beispielsweise NaOH, KOH, Ca(OH)₂), Carboxylate (beispielsweise Natrium- oder Kaliumsalze von C₁-C₄-Alkylearbonsäuren oder CICH₂COONa), Oxide (beispielsweise CaO, MgO, ZnO, Tl₂O₃, PbO), Phosphine (beispielsweise PPh₃), Zinksalze (ZnCl₂) und Ionentauscher (stark oder schwach alkalische Anionentauscher, wie z.B. DOWEX® MSA-1).

Der Katalysator wird üblicherweise in Mengen 0,001 bis 0,3 mol%, bevorzugt 0,005 bis 0,25, besonders bevorzugt 0,01 bis 0,2 und ganz besonders bevorzugt 0,02 bis 0,1 mol% bezogen auf die Ausgangsverbindung (II) eingesetzt.

Die Reaktion wird erfindungsgemäß bei einer Temperatur von mindestens 40 °C, bevorzugt mindestens 50, besonders bevorzugt mindestens 60 und ganz besonders bevorzugt mindestens 70 °C durchgeführt.

Die Reaktionstemperatur liegt bevorzugt oberhalb der Siedetemperatur des abzutrennenden Alkohols.

Erfindungsgemäß beträgt die obere Grenze der Temperatur nicht mehr als 120 °C, bevorzugt nicht mehr als 110 °C.

Eine unkatalysierte Reaktion benötigt in der Regel mindestens 110 °C um akzeptable Umsätze zu erbringen, gute Umsätze werden in der Regel erst ab 120 bis 130 °C erzielt. Durch derartige hohe Temperaturen wird jedoch häufig ein gefärbtes Produkt erhalten. Besonders empfindliche Substrate, wie beispielsweise polymerisierbare Monoole oder Polyole, neigen bei Temperaturen oberhalb von 130 °C zur thermischen Polymerisation und waren daher mit den aus dem Stand der Technik bekannten thermischen Umsetzungen nicht herstellbar.

Mithin ist das erfindungsgemäße Verfahren mit Vorteil zur Herstellung von 1,3,5-Triazincarbamaten und -harnstoffen mit mindestens einer polymerisierbaren Gruppe anwendbar.

Werden polymerisierbare Verbindungen eingesetzt, kann die Reaktion bevorzugt in Gegenwart von Radikalstabilisatoren durchgeführt. Geeignete Radikalstabilisatoren sind dem Fachmann bekannt, bevorzugt sind 4-Methoxyphenol (100-4000 ppm), 2,6-Di-t-Butylhydrochinon (50-1000 ppm), Phenothiazin (10-500 ppm) oder Triphenylphosphit (50-1000 ppm).

Ein Vorteil der erfindungsgemäßen Reaktion ist, daß durch die Zugabe des erfindungsgemäßen Katalysators bei gleicher oder verkürzter Reaktionszeit und mindestens gleichen Umsätzen unter ansonsten gleichen Bedingungen um mindestens 10 °C, bevorzugt mindestens 15 und besonders bevorzugt mindestens 20 °C gegenüber der unkatalysierten Reaktion abgesenkt werden kann.

Die Reaktionszeit ist je nach Substrat unterschiedlich und kann von 15 min bis 12 Stunden betragen, bevorzugt 30 min bis 10 Stunden, besonders bevorzugt 45 min bis 8 Stunden und ganz besonders bevorzugt 1 bis 7 Stunden.

Die Stöchiometrie bzgl. eingesetztem Alkohol R¹OH, R²OH und R³OH bzw. Amin R¹NH₂, R²NH₂ und R³NH₂ im Verhältnis zu umzusetzenden Carbamatgruppen beträgt in der Regel 0,5 -1,5 : 1 mol/mol, bevorzugt 0,7-1,3 : 1, besonders bevorzugt 0,8 bis 1,2: 1, ganz besonders bevorzugt 0,8 - 1,1 : 1, insbesondere 0,9 - 1 :1 und speziell 0,95-1,0 :1 mol/mol.

Die Reaktion kann in Substanz oder in einem geeigneten Lösungsmittel erfolgen, d.h. einem Lösungsmittel, das nicht mit einem 1,3,5-Triazincarbamat oder -harnstoff reagiert. Dies können beispielsweise sein Aceton, Acetylaceton, Acetoessigester, Ethylacetat, Butylacetat, Ethylenglycoldimethylether, Ethylenglycoldiethylether, Ethylenglycoldi-n-butylether, Diethylenglycoldimethylether, Diethylenglycoldiethylether, Diethylenglycoldi-n-butylether, C₁-C₄-Alkylencarbonate, insbesondere Ethylencarbonat, 1,2- oder 1,3-Propylencarbonat , THF, Dioxan, Dimethylformamid, Dimethylsulfoxid, N-Methylpyrrolidon, 1,3-Dioxolan, *iso*-Butyl-methylketon, Ethylmethylketon, Diethylether, tert-Butylmethylether, tert-Butylethylether, n-Pentan, n-Hexan, n-Heptan, Cyclohexan, Methylcyclohexan, Benzol, Toluol, Xylol, Paraffine, Naphta, Mineralöl oder Petroletherfraktionen.

Bevorzugt wird die Reaktion in Substanz durchgeführt.

Die Umsätze, die mit dem erfindungsgemäßen Verfahren erzielt werden, betragen in der Regel mindestens 40 %, bevorzugt mindestens 50 %, besonders bevorzugt mindestens 70 und ganz besonders bevorzugt mindestens 80 %.

Die Reaktion kann in einem unter den Reaktionsbedingungen inerten Gas oder Gasgemisch durchgeführt, beispielsweise solche mit einem Sauerstoffgehalt unter 10, bevorzugt unter 8, besonders bevorzugt unter 7 Vol%, bevorzugt sind Stickstoff, Argon, Helium, Stickstoff - Edelgas - Gemische, Kohlenstoffdi- oder -monooxid, besonders bevorzugt ist Stickstoff.

In einer bevorzugten Ausführungsform des erfindungsgemäßen Verfahrens werden die freigesetzten niederen Alkohole R⁴OH, R⁶OH, R⁶OH in geeigneter Weise abgetrennt, wodurch das sich einstellende Reaktionsgleichgewicht zugunsten des Produktes verschoben wird.

Die Abtrennung des niederen Alkohols R⁴OH, R⁵OH, R⁶OH kann beispielsweise erfolgen durch Destillation, Strippen, Vakuum, azeotrope Entfernung, Absorption, Pervaporation und Diffusion über Membranen.

Bevorzugt ist Abdestillieren, gegebenenfalls unter Vakuum, was gegebenenfalls durch Strippen mit einem unter den Reaktionsbedingungen inerten Gas unterstützt werden kann.

Zum Strippen wird ein unter den Reaktionsbedingungen inertes Gas oder Gasgemisch durch das Reaktionsgemisch geleitet, beispielsweise durch Einperlung.

Absorption kann beispielsweise mit Molekularsieben (Porengröße z.B. im Bereich von etwa 3-10 Angström) erfolgen. Diffusion kann beispielsweise mit Hilfe geeigneter semipermeabler Membranen erfolgen.

Die Reaktion kann erfindungsgemäß kontinuierlich, diskontinuierlich oder semikontinuierlich erfolgen, bevorzugt diskontinuierlich oder semikontinuierlich.

Beispielsweise wird dazu das Ausgangsmaterial der Formel (II) vorgelegt und auf die gewünschte Reaktionstemperatur gebracht.

Vor oder nach Erreichen der gewünschten Reaktionstemperatur kann der Katalysator zumindest teilweise zugegeben werden und mit der Alkohol/das Amin R¹XH, R²XH, R³XH vollständig, portionsweise oder kontinuierlich zugegeben werden. Falls der Katalysator noch nicht vollständig zugegeben worden ist, kann dieser ebenfalls portionsweise nachgegeben werden.

Es kann vorteilhaft sein, die Reaktionstemperatur im Verlauf der Reaktion zu steigern, beispielsweise um mindestens 10 °C, bevorzugt um mindestens 15 und besonders bevorzugt um mindestens 20 °C gegenüber der Temperatur zu Beginn der Reaktion.

Der Verlauf der Reaktion kann beispielsweise verfolgt werden, in dem man die Menge des freigesetzten Alkohols R⁴OH, R⁵OH und R⁶OH verfolgt und bei dem gewünschten Umsatz abbricht.

Die Reaktion kann beispielsweise durch Herunterkühlen oder durch direktes Abkühlen mit einem Lösungsmittel gestoppt werden.

Bevorzugt wird die Reaktion in einem rückvermischten Reaktionskessel durchgeführt, der beispielsweise durch Rühren, Eindüsen oder durch einen Umpumpkreislauf durchmischt werden kann.

Die Temperatureinstellung kann entweder über die Reaktorwände erfolgen oder durch einen im Umpumpkreislauf befindlichen Wärmetauscher.

Wird der freigesetzte niedere Alkohol R⁴OH, R⁵OH und R⁶OH durch Destillation und/oder Strippen abgetrennt, so kann dem Reaktor eine Packungs- oder Bodenkolonne aufgesetzt sein, für die 2 bis 10 theoretische Böden in der Regel ausreichend sind.

Zur Unterstützung der Abtrennung des niederen Alkohols kann ein leichtes Vakuum angelegt werden, beispielsweise kann die Reaktion bei einem Druck von 200 hPa bis Normaldruck, bevorzugt 300 hPa bis Normaldruck, besonders bevorzugt 500 hPa bis Normaldruck, ganz besonders bevorzugt bei 800 hPa bis Normaldruck und insbesondere bei Normaldruck durchgeführt werden.

Nach Beendigung der Reaktion kann das Reaktionsgemisch noch einer Wäsche und/oder Entfärbung unterzogen werden,

Zur Wäsche wird das Reaktionsgemisch in einem Waschapparat mit einer Waschflüssigkeit, beispielsweise Wasser oder einer 5 - 30 Gew%-igen, bevorzugt 5 - 20, besonders bevorzugt 5 - 15 Gew%-igen Kochsalz-, Kaliumchlorid-, Ammoniumchlorid-, Natriumsulfat- oder Ammoniumsulfatlösung, bevorzugt Wasser oder Kochsalzlösung, behandelt.

Die Wäsche kann beispielsweise in einem Rührbehälter oder in anderen herkömmlichen Apparaturen, z.B. in einer Kolonne oder Mixer-Settler-Apparatur, durchgeführt werden.

Falls erforderlich kann das Reaktionsgemisch einer Entfärbung, beispielsweise durch Behandlung mit Aktivkohle oder Metalloxiden, wie z.B. Aluminiumoxid, Siliciumoxid, Magnesiumoxid, Zirkonoxid, Boroxid oder Gemischen davon, in Mengen von beispielsweise 0,1 - 50 Gew%, bevorzugt 0,5 bis 25 Gew%, besonders bevorzugt 1 - 10 Gew% bei Temperaturen von beispielsweise 10 bis 100 °C, bevorzugt 20 bis 80 °C und besonders bevorzugt 30 bis 60 °C unterworfen werden.

Dies kann durch Zugabe des pulver- oder granulatförmigen Entfärbungsmittels zum Reaktionsgemisch und nachfolgender Filtration oder durch Überleiten des Reaktionsgemisches über eine Schüttung des Entfärbungsmittels in Form beliebiger, geeigneter Formkörper erfolgen.

Ein Vorteil der vorliegenden Erfindung besteht darin, daß durch die erfindungsgemäße katalysierte Verfahrensführung weniger im Ausgangsprodukt enthaltene Carbamatgruppen -COOR⁴, -COOR⁵ bzw. -COOR⁶ hydrolysiert werden, als in der rein thermischen Verfahrensführung und somit bei Einsatz von 1,3,5-Triazin-tris-carbamaten als Ausgangsprodukte im erfindungsgemäßen katalysierten Verfahrensführung ein geringerer Anteil an 2-Amino-1,3,5-Triazin-4,6-biscarbamaten erhalten wird, als bei der thermischen, unkatalysierten Reaktionsführung. Diese 2-Amino-1,3,5-Triazin-4,6-bis-carbamate neigen zur Kristallisation, führen daher also zu Ausfällungen im Produkt, und können zu optischen Defekten führen, wenn die so verunreinigten Produkte in einer Lackbeschichtung eingesetzt werden.

Die nach dem erfindungsgemäßen Verfahren hergestellten 1,3,5-Triazincarbamate können verwendet werden zur Beschichtung von verschiedenen Substraten, wie z. B. Holz, Holzfurnier, Papier, Pappe, Karton, Textil, Leder, Vlies, Kunststoffoberflächen, Glas, Keramik, mineralische Baustoffe, Metalle oder beschichtete Metalle.

Bei einer Verwendung in Beschichtungsmittel können die erfindungsgemäß hergestellten 1,3,5-Triazincarbamate insbesondere in Grundierungen, Füllern, pigmentierten Decklacken und Klarlacken im Bereich Autoreparatur- oder Großfahrzeuglackierung eingesetzt werden. Besonders geeignet sind solche Beschichtungsmittel für Anwendungen, in denen eine besonders hohe Applikationssicherheit, Außenwitterungsbeständigkeit, Optik, Lösemittel-, Chemikalien- und Wasserfestigkeit gefordert werden, wie in der Autoreparatur- und Großfahrzeuglackierung.

Die Beschichtungen können thermisch und/oder - wenn sie radikalisch polymerisierbare Gruppen enthalten - durch aktinische Strahlung gehärtet werden.

Die Aushärtung der Beschichtung durch aktinische Strahlung kann die Verwendung eines Photoinitiators erfordern.

Für die thermische Härtung der Beschichtungen werden der Formulierung Polyolkompenten zugesetzt, so dass eine Vernetzung eintritt.

In dieser Schrift verwendete ppm- und Prozentangaben beziehen sich, falls nicht anders angegeben, auf Gewichtsprozente und -ppm.

Die folgenden Beispiele sollen die Erfindung erläutern, aber nicht auf diese Beispiele einschränken.

### Beispiele

### Vergleichsbeispiel 1

In 100 ml n-Butanol wurden 1 g 2,4,6-Tris(methylcarbamoyl)-1,3,5-triazin gelöst und bei 110 °C gerührt.

Umsatz nach 280 Minuten: 78 % 2,4,6-Tris(butylcarbamoyl)-1,3,5-triazin. Der Umsatz an 2,4,6-Tris(butylcarbamoyl)-1,3,5-triazin wurde über HPLC bestimmt.

### Beispiel 1

In einem 250-ml-Vierhals-Reaktionskolben, ausgestattet mit Destillierbrücke und Liebigkühler und Rührer, wurden 40,5 g n-Butanol vorgelegt und 0,5 ml butanolische Cäsiumacetatlösung (2,5 mg/l) zudosiert.

Nach Erreichen der Innentemperatur von 110 °C wurden 2,50 g 2,4,6-Tris(methylcarbamoyl)-1,3,5-triazin eingerührt und aufgelöst.

Das entstehende Methanol wurde abdestilliert.

Umsatz nach 280 Minuten: 85 % 2,4,6-Tris(butylcarbamoyl)-1,3,5-triazin (HPLC).

### Vergleichsbeispiel 2

In einem 250-ml-Vierhals-Reaktionskolben, ausgestattet mit Destillierbrücke und Liebigkühler und Rührer, wurden in 4,74 g n-Butylacetat 6,0 g 2,4,6-Tris(methylcarbamoyl)-1,3,5-triazin, 6,97 g 2-Hydroxyethylacrylat, 12,5 mg 4-Methoxyphenol, 4 mg 2,6-Di-t-butyl-p-kresol und 0,3 mg Phenothiazin gelöst und auf eine Innentemperatur von 110 °C gebracht. Das entstehende Methanol wurde abdestilliert.

Umsatz nach 300 Minuten: 25 % des 2-Hydroxyethylacrylats, 4 % 2,4,6-Tris(2-ethoxyacrylato-carbamoyl)-1,3,5-triazin (HPLC).

### Beispiel 2

In einem 250-ml-Vierhals-Reaktionskolben, ausgestattet mit Destillierbrücke und Liebigkühler und Rührer, wurden in 12,32 g n-Butylacetat 6,0 g 2,4,6-Tris(methylcarbamoyl)-1,3,5-triazin, 6,97 g 2-Hydroxyethylacrylat, 12,5 mg 4-Methoxyphenol, 4 mg 2,6-Di-t-butyl-p-kresol und 0,3 mg Phenothiazin sowie 0,96 mg Cäsiumacetat gelöst und auf eine Innentemperatur von 110 °C gebracht. Das entstehende Methanol wurde abdestilliert.

Umsatz nach 300 Minuten: 50 % des 2-Hydroxyethylacrylats, 17 % 2,4,6-Tris(2-ethoxy-acrylato-carbamoyl)-1,3,5-triazin (HPLC).

### Beispiele 3-10

Eine Suspension aus 0,136 g p-Methoxyphenol, 0,045 g di-tert-Butyl-p-kresol, 0,003 g Phenothiazin, 0,016 g Dibutylzinndilaurat (DBTL), 2,4,6-Trisalkoxycarbamoyl-1,3,5-triazin, Diol und/oder Polyesterol sowie Hydroxyethylacrylat (HEA) wie in der Tabelle angegeben in 30,0 ml Methylisobutylketon (MIBK) wurde 4 Stunden bei 112 °C Badtemperatur gerührt. Anschließend wurde die Reaktionsmischung bei 52 °C Badtemperatur und einem Vakuum von 750 mbar 2 Stunden destilliert. Es wurde eine klare Harzlösung erhalten. Die eingesetzten molaren Mengen sind der Tabelle zu entnehmen.

### Filmprüfungen

Die Harzlösungen wurden durch Zugabe von Methylisobutylketon auf eine Viskosität von ca. 3,5 Pas eingestellt und mit 4 Gewichtsprozent (bezogen auf den Feststoffgehalt) 2-Hydroxy-2-methyl-1-phenyl-propan-1-on als Photoinitiator (Darocur^{®} 1173 der Firma Ciba Spezialitätenchemie) gemischt. Die Beschichtungsmassen wurden mit Hilfe einer Kastenrakel auf das jeweilige Substrat aufgetragen und zum Entfernen des Lösemittels 30 Minuten bei 60 °C getrocknet.

Die Beschichtungen wurden durch 30 minütiges Tempern thermisch gehärtet oder unter einer undotierten Quecksilberhochdrucklampe (Leistung 120 W/cm) mit einem Lampenabstand zum Substrat von 12 cm und einer Bandgeschwindigkeit von 10 m/min etwa bei einer Temperatur von 100 °C belichtet oder zuerst belichtet und anschließend thermisch gehärtet.

Die Pendelhärte (PD) wurde nach DIN 53157 bestimmt und ist ein Maß für die Härte der Beschichtung. Die Angabe erfolgt in Sekunden bis zum Stillstand des Pendels (s). Hohe Werte bedeuten dabei hohe Härte. Die Filme zur Bestimmung der Pendelhärte wurden mit Hilfe einer Kastenrakel auf Glas aufgetragen. Die Schichtdicke vor der Härtung betrug 100 µm.

Die Erichsentiefung (ET) wurde nach DIN 53156 bestimmt und ist ein Maß für die Flexibilität und Elastizität. Die Angabe erfolgt in Millimeter (mm). Hohe Werte bedeuten hohe Flexibilität. Die Filme zur Bestimmung der Erichsentiefung wurden mit Hilfe einer Spiralrakel auf Blech aufgetragen. Die Schichtdicke vor der Härtung beträgt 50 µm.

**Tabelle 1**

| Beispiel | 2,4,6-Tris(alkoxycarbamoyl)-1,3,5-triazin (mol) | HEA | Diol | Härtung | PD (s) | ET (mm) |
|---|---|---|---|---|---|---|
| | | (mol) | (mol) | | | |
| | | | | therm. | 8 | 9,8 |
| 3 | 0,4 ¹⁾ | 1,2 | 0,2 A | photochem. | 10 | 9,9 |
| | | | | photochem.+therm. | 39 | 9,8 |
| | | | | therm. | 17 | 9,8 |
| 4 | 0,2 ¹⁾ | 0,6 | 0,05 A | photochem. | 21 | 9,8 |
| | | | | photochem.+therm. | 76 | 9,4 |
| | | | | therm. | 54 | 9,7 |
| 5 | 0,1 ¹⁾ | 0,6 | 0,025 A | photochem. | 36 | 7,2 |
| | | | | photochem.+therm. | 170 | 7,3 |
| | | | | therm. | 46 | 5,1 |
| 6 | 0,1 ²⁾ | 0,3 | - | photochem. | 200 | 1,2 |
| | | | | photochem.+therm. | 235 | 2,1 |
| | | | | therm. | 60 | 9,8 |
| 7 | 0,1 ²⁾ | 0,3 | 0,125 A | photochem. | 83 | 6,1 |
| | | | | photochem.+therm. | 182 | 1,1 |
| | | | | therm. | 126 | 5,1 |
| 8 | 0,4 ²⁾ | 1,2 | 0,008 A | photochem. | 153 | 1,1 |
| | | | | photochem.+therm. | 235 | 3,8 |
| | | | | therm. | 221 | 3,5 |
| 9 | 0,4 ²⁾ | 1,2 | 0,2 B | photochem. | 161 | 2,3 |
| | | | | photochem.+therm. | 242 | 2,1 |
| | | | 6,7 A | therm. | 66 | 4,5 |
| 10 | 0,1 ²⁾ | 0,3 | 0,0067 B | photochem. | 129 | 1,1 |
| | | | | photochem.+therm. | 210 | 1,1 |

| | | | | | | |
|---|---|---|---|---|---|---|
| 1) 2,4,6-Tris(methoxycarbamoyl)-1,3,5-triazin 2) 2,4,6-Tris(methoxy/butoxy-carbamoyl)-1,3,5-triazin (molares Verhältnis Methyl:Butyl=60:40) A Polyester aus 1 mol Adipinsäure, 1 mol Isophthalsäure und 2 mol 1,6-Hexandiol, Molmasse ca. 1000 g/mol B 1,4-Butandiol | | | | | | |

Die Beispiele zeigen, dass eine vollständige Härtung erst durch Kombination von thermischer und photochemischer Vernetzung erreicht wird. Auf diese Weise erhält man mit den erfindungsgemäßen Verbindungen außergewöhnlich harte Beschichtungen.

## Patentansprüche

1. Verfahren zur Herstellung von 1,3,5-Triazincarbamaten der Formel (1), 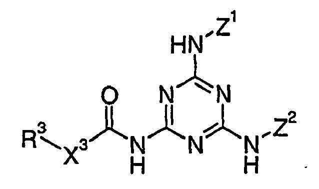 aus 1,3,5-Triazincarbamaten der Formel (II), 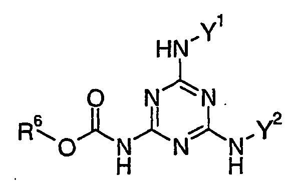 worin
Y¹ und Z¹ entweder beide Wasserstoff oder Y¹ eine Gruppe der Formel-(CO)-O-R⁴ und Z¹ eine Gruppe der Formel-(CO)-X¹-R¹,
Y² und Z² entweder beide Wasserstoff oder Y² eine Gruppe der Formel-(CO)-O-R⁵ und Z² eine Gruppe der Formel -(CO)-X²-R²,
R¹, R², R³, R⁴, R⁵ und R⁶ jeweils unabhängig voneinander den Rest eines Alkohols oder Amins und
X¹, X² und X³ jeweils unabhängig voneinander Sauerstoff oder unsubstituierten Stickstoff (NH)
bedeuten,
**dadurch gekennzeichnet, dass**
man die Reaktion bei einer Temperatur von 40 bis 120 °C und
in Gegenwart mindestens eines Katalysators durchführt, der ausgewählt ist aus der Gruppe umfassend Zinnverbindungen, Cäsiumsalze, Alkalimetall(hydrogen)-carbonate und tertiäre Amine.

2. Verfahren nach Anspruch 1, **dadurch gekennzeichnet, daß** die Temperatur zwischen 60 und 110 °C beträgt.

3. Verfahren nach einem der vorstehenen Ansprüche, **dadurch gekennzeichnet, daß** es sich bei den Resten R¹, R² und R³ unabhängig voneinander um C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können,
sowie ferner um Reste
-(CO)-R⁷, -(CO)-O-R⁷ oder -(CO)-(NH)-R⁷,
worin
R⁷ C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können bedeuten kann,
handelt.

4. Verfahren nach einem der vorstehenen Ansprüche, **dadurch gekennzeichnet, daß** die Alkohole R¹OH, R²OH und R³OH bzw. Amine R¹NH₂, R²NH₂ und R³NH₂, eine Siedepunktsdifferenz von mindestens 20 °C gegenüber dem höchstsiedenden der Alkohole R⁴OH, R⁵OH und R⁶OH aufweisen.

5. Verfahren nach einem der vorstehenen Ansprüche, **dadurch gekennzeichnet, daß** es sich bei mindestens einem der Alkohole R¹OH, R²OH und R³OH um ein alkoxyliertes Monool der Formel
R⁸-O-[-Xᵢ-]ₙ-H
handelt, worin
R⁸ C₁ - C₁₈-Alkyl sein kann,
n eine positive ganze Zahl zwischen 1 und 50 und
jedes Xᵢ für i = 1 bis n unabhängig voneinander ausgewählt sein kann aus der Gruppe -CH₂-CH₂-O-, -CH₂-CH(CH₃)-O-, -CH(CH₃)-CH₂-O-, -CH₂-C(CH₃)₂-O-, -C(CH₃)₂-CH₂-O-, -CH₂-CHVin-O-, -CHVin-CH₂-O-, -CH₂-CHPh-O- und -CHPh-CH₂-O-,
worin Ph für Phenyl und Vin für Vinyl steht.

6. Verfahren nach einem der vorstehenen Ansprüche, **dadurch gekennzeichnet, daß** es sich bei mindestens einem der Alkohole R¹OH, R²OH und R³OH um ein Monool handelt, das mindestens eine polymerisierbare Gruppe und genau eine Hydroxygruppe trägt.

7. Verfahren nach Anspruch 6, **dadurch gekennzeichnet, daß** es sich bei den Verbindungen, die mindestens eine polymerisierbare Gruppe und genau eine Hydroxygruppe tragen um solche der Formeln
(III) H₂C=CR⁹-CO-O-R¹⁰-OH,
(IV) H₂C=CR⁹-CO-O-[-Xᵢ-]ₖ-H
oder
(V) H₂C=CH-O-R¹⁰-OH
handelt,
worin
R⁹ Wasserstoff oder Methyl, bevorzugt Wasserstoff,
R¹⁰ einen zweiwertigen linearen oder verzweigten C₂-C₁₈-Alkylenrest,
Xᵢ die gleiche Bedeutung hat wie in Anspruch 5 ausgeführt und
k eine positive ganze Zahl von 1 bis 20
bedeutet.

8. Verfahren nach einem der Ansprüche 6 oder 7, **dadurch gekennzeichnet, daß** mindestens einer der Alkohole R¹OH, R²OH und R³OH ausgewählt ist unter Polyetherolen oder Polyesterolen unter der Voraussetzung, daß gleichzeitig mindestens einer der Alkohole R¹OH, R²OH und R³OH ein Monool mit mindestens einer polymerisierbaren Gruppe und genau einer Hydroxygruppe ist.

9. Verfahren nach einem der vorstehenden Ansprüche, **dadurch gekennzeichnet, daß** die niederen Alkohole R⁴OH, R⁵OH und R⁶OH destillativ aus dem Reaktionsgemisch abgetrennt werden.

10. Verwendung von 1,3,5-Triazincarbamaten und -harnstoffen hergestellt nach einem Verfahren gemäß einem der vorstehenden Ansprüche in der Beschichtung von Substraten ausgewählt aus der Gruppe umfassend Holz, Holzfurnier, Papier, Pappe, Karton, Textil, Leder, Vlies, Kunststoffoberflächen, Glas, Keramik, mineralische Baustoffe, Metalle und beschichtete Metalle.

## Claims

1. A process for preparing a 1,3,5-triazine carbamate of formula (1), 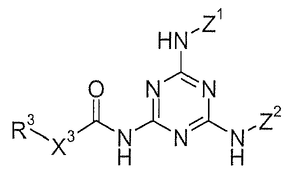 from a 1,3,5-triazine carbamate of formula (ii), 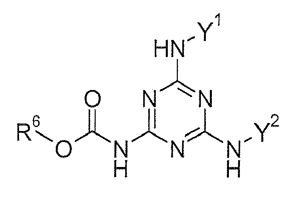 in which
either Y¹ and Z¹ are both hydrogen or Y¹ is a group of formula -(CO)-O-R⁴ and Z¹ is a group of formula -(CO)-X¹-R¹,
either Y² and Z² are both hydrogen or Y² is a group of formula -(CO)-O-R⁵ and Z² is a group of formula -(CO)-X²-R²,
R¹, R², R³, R⁴, R⁵ and R⁶ each independently of one another are the radical of an alcohol or amine and
X¹, X² and X³ each independently of one another are oxygen or unsubstituted nitrogen (NH),
which comprises
conducting the reaction at a temperature of 40 to 120°C and
in the presence of at least one catalyst selected from the group comprising tin compounds, cesium salts, alkali metal (hydrogen)carbonates and tertiary amines.

2. The process according to claim 1, wherein the temperature is between 60 and 110°C.

3. The process according to either of the preceding claims, wherein the radicals R¹, R² and R³ independently of one another are C₁ - C₁₈ alkyl, C₂ - C₁₈ alkyl, interrupted if appropriate by one or more oxygen and/or sulfur atoms and/or by one or more substituted or unsubstituted imino groups, or are C₂ - C₁₈ alkenyl, C₆ - C₁₂ aryl, C₅ - C₁₂ cycloalkyl or a five- or six-membered heterocycle containing oxygen, nitrogen and/or sulfur atoms, it being possible for said radicals each to be substituted by aryl, alkyl, aryloxy, alkyloxy, heteroatoms and/or heterocycles, or else are radicals
-(CO)-R⁷, -(CO)-O-R⁷ or -(CO)-(NH)-R⁷,
in which
R⁷ can be C₁ - C₁₈ alkyl, C₂ - C₁₈ alkyl, interrupted if appropriate by one or more oxygen and/or sulfur atoms and/or by one or more substituted or unsubstituted imino groups, or can be C₂ - C₁₈ alkenyl, C₆ - C₁₂ aryl, C₅ - C₁₂ cycloalkyl or a five- or six-membered heterocycle containing oxygen, nitrogen and/or sulfur atoms, it being possible for said radicals each to be substituted by aryl, alkyl, aryloxy, alkyloxy, heteroatoms and/or heterocycles.

4. The process according to any one of the preceding claims, wherein the alcohols R¹OH, R²OH and R³OH and/or amines R¹NH₂, R²NH₂ and R³NH₂, have a boiling point difference of at least 20°C from the highest-boiling of the alcohols R⁴OH, R⁵OH and R⁶OH.

5. The process according to any one of the preceding claims, wherein at least one of the alcohols R¹OH, R²OH and R³OH is an alkoxylated monool of formula
R⁸-O-[-Xᵢ-]ₙ-H
in which
R⁸ can be C₁ - C₁₈ alkyl,
n is a positive integer between 1 and 50 and
each Xᵢ for i = 1 to n can be selected independently of the others from the group consisting of -CH₂-CH₂-O-, -CH₂-CH(CH₃)-O-, -CH(CH₃)-CH₂-O-, -CH₂-C(CH₃)₂-O-, -C(CH₃)₂-CH₂-O-, -CH₂-CHVin-O-, -CHVin-CH₂-O-, -CH₂-CHPh-O- and -CHPh-CH₂-O-,
in which Ph is phenyl and Vin is vinyl.

6. The process according to any one of the preceding claims, wherein at least one of the alcohols R¹OH, R²OH and R³OH is a monool which carries at least one polymerizable group and exactly one hydroxyl group.

7. The process according to claim 6, wherein the compounds which carry at least one polymerizable group and precisely one hydroxyl group are compounds of formula
(III) H₂C=C⁹-CO-O-R¹⁰-OH,
(IV) H₂C=CR⁹-CO-O-[-Xᵢ-]ₖ-H
or
(V) H₂C=CH-O-R¹⁰-OH
in which
R⁹ is hydrogen or methyl, preferably hydrogen,
R¹⁰ is a divalent linear or branched C₂-C₁₈ alkylene radical,
Xᵢ has the same definition as set out in claim 5 and
k is a positive integer from 1 to 20.

8. The process according to either of claims 6 and 7, wherein at least one of the alcohols R¹OH, R²OH and R³OH is selected from polyetherols or polyesterols with the proviso that at the same time at least one of the alcohols R¹OH, R²OH and R³OH is a monool containing at least one polymerizable group and precisely one hydroxyl group.

9. The process according to any one of the preceding claims, wherein the lower alcohols R⁴OH, R⁵OH and R⁶OH are separated by distillation from the reaction mixture.

10. The use of a 1,3,5-triazine carbamate or 1,3,5-triazine urea prepared by a process according to any one of the preceding claims in the coating of substrates selected from the group comprising wood, wood veneer, paper, paper board, cardboard, textile, leather, nonwoven fabric, plastics surfaces, glass, ceramic, mineral building materials, and coated and uncoated metals.

## Revendications

1. Procédé de préparation de 1,3,5-triazine carbamates de formule (I), 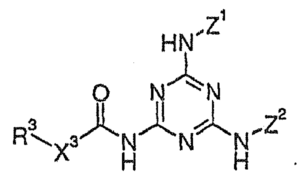 à partir de 1,3,5-triazine carbamates de formule (II), 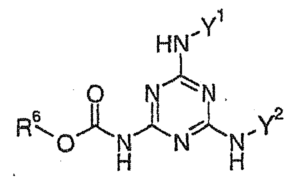 dans lesquelles :
Y¹ et Z¹ signifient soit tous deux hydrogène, soit Y¹ un groupe de formule -(CO)-O-R⁴ et Z¹ un groupe de formule -(CO)-X¹-R¹,
Y² et Z² signifient soit tous deux hydrogène, soit Y² un groupe de formule -(CO)-O-R⁵ et Z² un groupe de formule -(CO)X²-R²,
R¹, R², R³, R⁴, R⁵ et R⁶ signifient chacun indépendamment les uns des autres le radical d'un alcool ou d'une amine, et
X¹, X² et X³ signifient chacun indépendamment les uns des autres un oxygène ou un azote non substitué (NH)
**caractérisé en ce que** :
on effectue la réaction à une température de 40 à 120°C et ce, en présence d'au moins un catalyseur sélectionné parmi le groupe comprenant des composés d'étain, des sels de césium, des (hydrogéno)carbonates de métal alcalin et des amines tertiaires.

2. Procédé selon la revendication 1, **caractérisé en ce que** la température est située entre 60 et 110°C.

3. Procédé selon l'une des revendications précédentes, **caractérisé en ce qu'**il s'agit, pour les radicaux R¹, R² et R³, indépendamment les uns des autres, d'un alkyle en C₁-C₁₈, d'un alkyle en C₂-C₁₈ éventuellement interrompu par un ou plusieurs atomes d'oxygène et/ou de soufre et/ou un ou plusieurs groupes imino substitués ou non substitués, d'un alcényle en C₂-C₁₈, d'un aryle en C₆-C₁₂, d'un cycloalkyle en C₅-C₁₂ ou d'un hétérocycle de cinq à six membres présentant des atomes d'oxygène, d'azote et/ou de soufre, les radicaux mentionnés pouvant être chaque fois substitués par un aryle, un alkyle, un aryloxy, un alkyloxy, des hétéroatomes et/ou des hétérocycles,
ainsi qu'en outre de radicaux
-(CO)-R⁷, -(CO)-O-R⁷ou -(CO)-(NH)-R⁷,
dans lesquels :
R⁷ peut signifier un alkyle en C₁-C₁₈, un alkyle en C₂-C₁₈ éventuellement interrompu par un ou plusieurs atomes d'oxygène et/ou de soufre et/ou un ou plusieurs groupes imino substitués ou non substitués, un alcényle en C₂-C₁₈, un aryle en C₆-C₁₂, un cycloalkyle en C₅-C₁₂ ou un hétérocycle de cinq à six membres présentant des atomes d'oxygène, d'azote et/ou de soufre, les radicaux mentionnés pouvant être chaque fois substitués par un aryle, un alkyle, un aryloxy, un alkyloxy, des hétéroatomes et/ou des hétérocycles.

4. Procédé selon l'une des revendications précédentes, **caractérisé en ce que** les alcools R¹OH, R²OH et R³OH ou, selon le cas, les amines R¹NH₂, R²NH₂ et R³NH₂ présentent une différence de point d'ébullition d'au moins 20°C par rapport à celui des alcools R⁴OH, R⁵OH et R⁶OH qui a le point d'ébullition le plus élevé.

5. Procédé selon l'une des revendications précédentes, **caractérisé en ce qu'**il s'agit pour au moins un des alcools R¹OH, R²OH et R³OH, d'un mono-ol alcoxylé de formule
R⁸-O-[-Xᵢ-]ₙ-H
dans laquelle :
R⁸ peut être un alkyle en C₁-C₁₈,
n est un nombre entier positif entre 1 et 50, et
chaque Xᵢ pour i=1 à n indépendamment l'un de l'autre peut être choisi parmi le groupe comprenant -CH₂-CH₂-O-, -CH₂-CH(CH₃)-O-, -CH(CH₃)-CH₂-O-, -CH₂-C(CH₃)₂-O-, -C(CH₃)₂-CH₂-O-, -CH₂-CHVin-O-, -CHVin-CH₂-O-, -CH₂-CHPh-O- et -CHPh-CH₂-O-,
dans lesquels Ph représente phényle et Vin, vinyle.

6. Procédé selon l'une des revendications précédentes, **caractérisé en ce qu'**il s'agit pour au moins un des alcools R¹OH, R²OH et R³OH d'un mono-ol qui porte au moins un groupe polymérisable et précisément un groupe hydroxy.

7. Procédé selon la revendication 6, **caractérisé en ce qu'**il s'agit, pour les composés qui portent au moins un groupe polymérisable et précisément un groupe hydroxy, de ceux de formules :
(III) H₂C=CR⁹-CO-O-R¹⁰-OH,
(IV) H₂C=CR⁹-CO-O-[-Xᵢ-]ₖ-H
ou
(V) H₂C=CH-O-R¹⁰-OH
dans lesquelles :
R⁹ signifie hydrogène ou méthyle, de préférence hydrogène,
R¹⁰ représente un radical alkylène en C₂-C₁₈ divalent linéaire ou ramifié,
Xᵢ a la même signification qu'à la revendication 5 et
k représente un nombre entier positif de 1 à 20.

8. Procédé selon l'une des revendications 6 ou 7, **caractérisé en ce qu'**au moins un des alcools R¹OH, R²OH et R³OH est choisi parmi les polyéthérols ou polyestérols à la condition qu'en même temps au moins un des alcools R¹OH, R²OH et R³OH soit un mono-ol avec au moins un groupe polymérisable et précisément un groupe hydroxy.

9. Procédé selon l'une des revendications précédentes, **caractérisé en ce que** les alcools inférieurs R⁴OH, R⁵OH et R⁶OH sont séparés du mélange réactionnel par distillation.

10. Utilisation de 1,3,5-triazine carbamates et urées préparés selon un procédé conforme à l'une des revendications précédentes dans le revêtement de substrats sélectionnés parmi le groupe comprenant le bois, le contreplacage en bois, le papier, le carton-pâte, le carton, le textile, le cuir, le non-tissé, des surfaces synthétiques, le verre, la céramique, des matières premières minérales, des métaux et des métaux revêtus.
